# EUROPEAN PATENT APPLICATION

(11) **EP 4 454 756 A1**
(43) Date of publication of application: **30.10.2024**
(21) Application number: 22911983.9
(22) Date of filing: 22.12.2022
(51) Int. Cl.: B01J 37/10, B01J 37/34, B01J 35/00, B01J 37/00, B01J 35/02, B01J 21/06, B01D 53/86

(54) **PHOTOCATALYST GRANULAR MATERIAL AND PREPARATION METHOD THEREFOR**

(30) Priority: 22.12.2021 KR 20210184890
(71) Applicant: Korea Institute of Civil Engineering and Building Technology, Goyang-si, Gyeonggi-do 10223 (KR); Bentech Frontier Co., Ltd, Gwangju 61186 (KR)
(72) Inventor: KOO, Hyun-Bon, Goyang-si Gyeonggi-do 10218 (KR); KWARK, Jong-Won, Goyang-si Gyeonggi-do 10356 (KR); KIM, Seong-Jun, Goyang-si Gyeonggi-do 10377 (KR); KI, Yun-jong, Jangseong-gun Jeollanam-do 57248 (KR)
(74) Representative: M. Zardi & Co S.A.
(86) International application number: PCT/KR2022/021082
(87) International publication number: WO 2023/121350

(57) **Abstract**

Disclosed are a photocatalytic reforming method, a photocatalyst granular material comprising photocatalyst crystals reformed thereby, and a preparation method therefor, the photocatalytic reforming method comprising the steps of: in order to separate a monatomic layer of a photocatalyst, hydrothermally synthesizing the photocatalyst by reacting the photocatalyst with an alkaline solution at a temperature of at least 100°C; and processing the hydrothermally synthesized photocatalyst so that crystals of the photocatalyst have tubular shapes.

## Description

### [Technical Field]

The present disclosure relates to a photocatalyst granular material and a preparation method therefor. One embodiment of the present disclosure relates to a photocatalyst granular material, capable of performing at least one of organic matter decomposition, bacteria removal, and virus removal, and a method for preparing the same.

This research was supported by a research grant from the "Photocatalytic reforming technology and air conditioning filter development" project of the 2021 KICT (Korea Institute of Civil Engineering and Building Technology) major program (Project No.: 20210218-001, Research Project Name: Construction industry innovation company support and performance expansion, Contribution rate: 1/2) and the "Development of emergency modular system for virus disaster response_Development of modular facility and quarantine system capable of preventing the spread of infectious diseases and enabling rapid response" project of the 2022 KICT major program (Project No.: 20220238-001, Research Project Name: Safety, security, land transportation technology development, Contribution rate: 1/2).

### [Background Art]

A large-scale outbreak of a new/variant virus could threaten people's lives and safety and cause serious damages to the national economy.

We are continuously and repeatedly experiencing shock and fear due to the introduction of new high-risk respiratory infectious diseases into the country in the 2000s, including SARS in 2003, swine flu in 2009, MERS in 2015, and COVID-19 in 2020. These respiratory infectious diseases have the characteristic of being spread when an infectious agent (pathogenic virus or bacteria) expelled by an infected person through coughing, etc. enters through the respiratory tract of an uninfected person. Actually, there are frequent cases where air in indoor living environments is contaminated by infectious agents and infectious diseases spread seriously. In addition, even from the perspective of economic damage, the scale of social and economic damages caused by MERS in 2015 is reported to be KRW 10.8448 trillion, and in particular, the scale of damages from COVID-19 is expected to be astronomical.

For this reason, interest in and needs for effective home quarantine technologies to respond to infectious diseases and create a safe and secure living environment are currently growing internationally. In fact, it is very important to manage contamination of infectious agents to suppress the spread of infectious diseases and minimize damage, in immigration facilities that are the entryway for overseas infectious diseases, hospital facilities where patients with infectious diseases cluster, elderly and nursing facilities where people vulnerable to infectious diseases live, educational and childcare facilities where infants and teenagers live together, public facilities and public transportation that unspecified numbers of people frequently enter and use, and the like.

In order to create a safe and secure living environment where the risk of spreading high-risk respiratory infectious diseases is minimized, a home quarantine technology that can effectively remove airborne infectious agents is needed. In particular, in order to minimize the risk of airborne spread of infectious diseases in hermetic spaces where sufficient ventilation is difficult due to fine dust problems, heating and cooling problems, etc., or where ventilation is impossible due to the special nature of space structure and use, there is a growing need to develop air purification devices (air purifiers, air sterilizers, purification and ventilation devices) with antiviral or antibacterial properties that can remove airborne infectious agents constantly, quickly, and directly.

### [Disclosure]

### [Technical Problem]

The present disclosure is intended to solve the problems of the related art described above, and one aspect of the present disclosure is to provide a photocatalyst granular material that has improved photocatalytic activity and increased adsorption capacity, compared to the related art, and allows decomposition and/or oxidization of organic matter and/or organisms, so as to exhibit a purifying function, an antiviral function, and an antibacterial function, by which harmful substances (fine dust precursors or volatile organic compounds) and harmful microorganisms (viruses or bacteria) can be eliminated, and a method of preparing the same.

However, technical problems to be solved in embodiments of the present disclosure are not limited to the problems to be solved, and other technical problems to be solved may exist.

### [Technical Solution]

As a technical means for achieving the above-described technical problem, a photocatalytic reforming method according to one aspect of the present disclosure may include performing hydrothermal synthesis to react a photocatalyst with an alkaline solution at a temperature of 100°C or higher so that a monoatomic layer of the photocatalyst is separated; and treating the hydrothermally synthesized photocatalyst so that a crystal of the photocatalyst has a tubular shape.

The photocatalyst according to one aspect of the present disclosure may include a plurality of photocatalyst crystals reformed by the photocatalytic reforming method according to the one aspect of the present disclosure.

The photocatalyst granular material according to one aspect of the present disclosure may include the photocatalyst according to the one aspect of the present disclosure.

A method for preparing a photocatalyst granular material according to one aspect of the present disclosure may include: preparing a photocatalyst reformed by the photocatalytic reforming method according to the one aspect of the present disclosure; producing a photocatalyst paste by mixing the reformed photocatalyst with distilled water; molding the photocatalyst paste into a granule (granular material); and drying the molded granule to form a photocatalyst granule.

A photocatalyst granular material preparation method according to another aspect of the present disclosure may include: preparing a photocatalyst reformed by the photocatalytic reforming method according to one aspect of the present disclosure; producing a photocatalyst paste by mixing the reformed photocatalyst with distilled water and further mixing an open-cell type circular foam made of a material with a lower melting point than that of the reformed photocatalyst or a piece of yarn made of a material with a lower melting point than that of the photocatalyst; molding the photocatalyst paste into a granule; and drying the molded granule to form a photocatalyst granule.

A method for preparing photocatalyst granular material according to still another aspect of the present disclosure may include: coating a granule with a liquid photocatalyst; and drying the granule coated with the liquid photocatalyst to form a photocatalyst granule, in which the coating the granule with the liquid photocatalyst may be to immerse the granule in the liquid photocatalyst or spray the liquid photocatalyst on the granule.

The solution to problem is merely illustrative and should not be construed as intended to limit the present disclosure. In addition to the embodiments described above, additional embodiments may be present in the drawings and detailed description of the disclosure.

### [Advantageous Effects]

According to the solution to problem described above, compared to the related art, a photocatalyst granular material may be implemented in which photocatalytic activity performance is improved, adsorption capacity is increased, an organic matter removal effect is increased by enabling decomposition and/or oxidization of organic matter and/or organisms, purification function, antiviral function, and antibacterial function may be exerted by inactivating and/or removing harmful microorganisms (viruses or bacteria), and a preparing process is simplified thereby.

### [Description of Drawings]

FIG. 1 is a conceptual view illustrating a photocatalytic reforming method according to one embodiment of the present disclosure.
FIG. 2 is an SEM photo of a photocatalyst (photocatalyst powder) before reforming (without being reformed) by the photocatalytic reforming method according to one embodiment of the present disclosure.
FIG. 3 is an SEM photo of Ti(Li-Ti) reformed by using Li according to the photocatalytic reforming method according to one embodiment of the present disclosure.
FIG. 4 is an SEM photo of Ti(Na-Ti) reformed by using Na according to the photocatalytic reforming method according to one embodiment of the present disclosure.
FIG. 5 is an SEM photo of Ti(K-Ti) reformed by using K according to the photocatalytic reforming method according to one embodiment of the present disclosure.
FIG. 6A is a table showing the measurement results of a surface area of a photocatalyst before and after reforming according to the photocatalytic reforming method according to one embodiment of the present disclosure.
FIG. 6B is a table showing the results of elemental analysis of a photocatalyst before and after reforming according to the photocatalytic reforming method according to one embodiment of the present disclosure.
FIG. 7A is a graph showing the analysis results of XRD patterns after drying three types of photocatalysts Li-Ti, Na-Ti, and K-Ti reformed by Li, Na, and K, respectively, according to the photocatalytic reforming method according to one embodiment of the present disclosure.
FIG. 7B is a graph showing the analysis results of XRD patterns after calcining three types of photocatalysts Li-Ti, Na-Ti, and K-Ti reformed by Li, Na, and K, respectively, according to the photocatalytic reforming method according to one embodiment of the present disclosure.
FIG. 8 is a graph showing the results of evaluating activities after calcining three types of photocatalysts Li-Ti, Na-Ti, and K-Ti reformed by Li, Na, and K, respectively, according to the photocatalytic reforming method according to one embodiment of the present disclosure.
FIG. 9 is a table summarizing the results of evaluating three types of photocatalysts Li-Ti, Na-Ti, and K-Ti reformed by Li, Na, and K, respectively, according to the photocatalytic reforming method according to one embodiment of the present disclosure.
FIG. 10 is a graph showing the results of evaluating organic matter removal performance of a photocatalyst that MTA powder has calcined after chemical treatment according to the photocatalytic reforming method according to one embodiment of the present disclosure (showing the results of photocatalyst activity evaluation for each treatment temperature).
FIG. 11 is a graph showing the results of analyzing XRD diffraction of a photocatalyst that MTA powder has calcined at a temperature of 550°C after chemical treatment according to the photocatalytic reforming method according to one embodiment of the present disclosure
FIG. 12 is a graph showing particle size distribution data related to primary pulverization of the photocatalytic reforming method according to one embodiment of the present disclosure.
FIG. 13 is a graph showing particle size distribution data related to secondary pulverization/classification of the photocatalytic reforming method according to one embodiment of the present disclosure.
FIG. 14 is a schematic flowchart illustrating a method for preparing a photocatalyst granular material according to a first embodiment of the present disclosure.
FIG. 15 is a table listing heating conditions according to a drying furnace type in the case of a continuous type drying furnace in step 5700 of the method for preparing the photocatalyst granular material according to the first embodiment of the present disclosure.
FIG. 16 is a table listing heating conditions according to a drying furnace type in the case of a non-continuous type drying furnace in step 5700 of the method for preparing the photocatalyst granular material according to the first embodiment of the present disclosure.
FIG. 17A is a schematic flowchart illustrating a method for preparing a photocatalyst granular material according to a second embodiment of the present disclosure in which photocatalyst paste is produced by mixing a photocatalyst with distilled water and an open-cell type circular foam.
FIG. 17B is a schematic flowchart illustrating a method for preparing a photocatalyst granular material according to a second embodiment of the present disclosure in which photocatalyst paste is produced by mixing a photocatalyst with distilled water and a yarn piece.
FIG. 18 is an exemplary view of the open-cell type foam of the method for preparing the photocatalyst granular material according to the second embodiment of the present disclosure.
FIG. 19 is a table listing heating conditions according to a drying furnace type in the case of a continuous type drying furnace in step 5700 of the method for preparing the photocatalyst granular material according to the second embodiment of the present disclosure.
FIG. 20 is a table listing heating conditions according to a drying furnace type in the case of a non-continuous type drying furnace in step 5700 of the method for preparing the photocatalyst granular material according to the second embodiment of the present disclosure.
FIG. 21 is a schematic flowchart illustrating a method for preparing a photocatalyst granular material according to a third embodiment of the present disclosure.
FIG. 22 is a table listing heating conditions according to a drying furnace type in the case of a continuous type drying furnace in step S300 of the method for preparing the photocatalyst granular material according to the third embodiment of the present disclosure.
FIG. 23 is a table listing heating conditions according to a drying furnace type in the case of a non-continuous type drying furnace in step S300 of the method for preparing the photocatalyst granular material according to the third embodiment of the present disclosure.
FIGS. 24 to 26 are graphs showing the results of evaluating bacteria and virus removal performances of a photocatalyst before reforming.
FIG. 27 is a graph showing the results of evaluating the virus removal performance of each reformed photocatalyst Li-Ti, Na-Ti, or K-Ti prepared by the photocatalytic reforming method according to one embodiment of the present disclosure.
FIGS. 28A to 30C show the results of evaluating the virus removal performances of photocatalyst granular materials prepared by the method for preparing the photocatalyst granular material according to the first to third embodiments of the present disclosure or photocatalyst granular materials according to the first to fourth embodiments of the present disclosure.
FIG. 31 is a graph for explaining effects according to a photocatalyst preparation step of the photocatalytic reforming method according to one embodiment of the present disclosure, specifically, a graph showing an amount of acetaldehyde removed for each calcination temperature.
FIG. 32 is a graph for explaining effects according to a photocatalyst preparation step of the photocatalytic reforming method according to one embodiment of the present disclosure, specifically, a graph showing an amount of carbon dioxide generated for each calcination temperature.
FIG. 33 is a graph for explaining effects according to a photocatalyst preparation step of the photocatalytic reforming method according to one embodiment of the present disclosure, specifically, a TEM measurement photo of prepared photocatalyst powder.
FIG. 34 shows XRD measurement results of photocatalyst powder prepared in the photocatalyst preparation step of the photocatalytic reforming method according to one embodiment of the present disclosure.
FIG. 35 shows an SEM/EDX (X1,300) photo of photocatalyst powder prepared in the photocatalyst preparation step of the photocatalytic reforming method according to one embodiment of the present disclosure.
FIG. 36 shows an SEM/EDX (X10,000) photo of photocatalyst powder prepared in the photocatalyst preparation step of the photocatalytic reforming method according to one embodiment of the present disclosure.

### [Best Mode]

Hereinafter, embodiments of the present disclosure will be described in detail, with reference to the accompanying drawings, so as to be easily embodied by those skilled in the art to which the present disclosure pertains. However, the present disclosure may be implemented in different forms and is not limited to the embodiments described herein. In order to clearly explain the present disclosure in the drawings, parts that are not related to the description are omitted, and similar parts are given similar reference numerals throughout the disclosure.

Throughout the present specification, when a part is said to be "connected" to another part, it includes not only cases where they are "directly connected," but also cases where they are "electrically connected" with another element interposed therebetween.

Throughout the present specification, when a member is said to be located "on," "above," "at the top of," "under," "below," or "at the bottom of" another member, it includes a case where a member is located on another member in contact with each other and a case where an intervening member is present between the two members.

Throughout the present specification, when a part "includes" a component, it means that other components may be further included, rather than excluding the other components, unless otherwise specified.

The present disclosure relates to a photocatalyst granular material and a preparation method therefor.

Hereinafter, for easy understanding of the present disclosure, a photocatalytic reforming method according to one embodiment of the present disclosure (hereinafter, referred to as 'a reforming method according to the present disclosure') will first be described.

The reforming method according to the present disclosure includes performing hydrothermal synthesis of reacting a photocatalyst with an alkaline solution at a temperature of at least 100°C so that a monoatomic layer of the photocatalyst is separated (First step). Additionally, the reforming method according to the present disclosure includes treating the hydrothermally synthesized photocatalyst so that photocatalyst crystals have a tubular shape (Second step). That is, in the reforming method according to the present disclosure, photocatalyst powder and the alkaline solution may be hydrothermally synthesized with each other such that the monoatomic layer is separated, and thereafter the photocatalyst crystal may be changed into the tubular shape.

As an example, in the first step, the photocatalyst and the alkaline solution may be mixed and reacted at a temperature of 100°C to 130°C.

As an example, the alkaline solution may contain at least one of Li, Na, and K.

Additionally, the photocatalyst may be anatase.

Also, the photocatalyst may be titanium oxide or metatitanic acid.

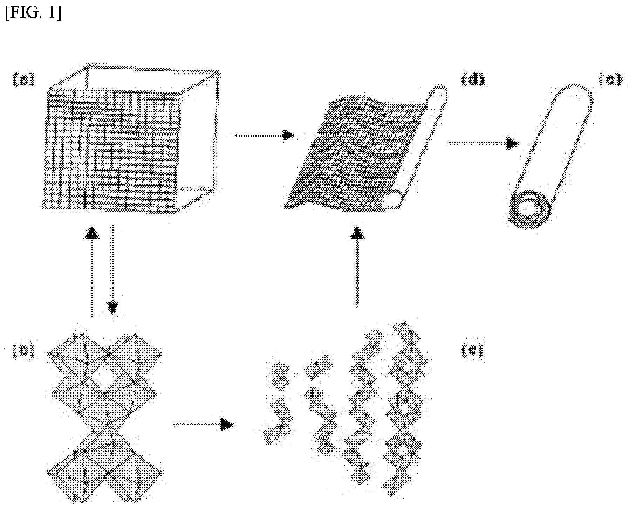

As described above, referring to FIG. 1, in the reforming method according to the present disclosure, photocatalyst (for example, titanium oxide) powder may be reacted in a high-temperature alkaline solution for a long time, and accordingly, the photocatalyst is separated into a monoatomic layer by the alkaline solution. The separated monoatomic layer may take the tubular shape as it is rolled. In this process, a dissolution process may be performed by an alkaline solution. Impurities contained in the photocatalyst may be removed during the dissolution process, thereby recovering only a pure photocatalyst. In the reforming method according to the present disclosure, the reaction was carried out by adding a general photocatalyst (titanium oxide), mixing it with an alkaline solution, and stirring the mixture. At this time, hydrothermal synthesis may be performed by maintaining the temperature of a reforming reactor at 130°C.

Additionally, in the present disclosure, preferably, the alkaline solution may contain at least one of Na and K. Specifically, the alkaline solution may contain at least one of sodium hydroxide (NaOH) and potassium hydroxide (KOH), and in the first step, the photocatalyst may be dispersed in the alkaline solution through energy equivalent to ultrasonic waves (i.e., dispersed by applying ultrasonic waves), and hydrothermal synthesis may be carried out by reacting the photocatalyst at a temperature of 100°C to 130°C for 15 to 20 hours. In addition, in the first step, after the hydrothermal synthesis, the photocatalyst may be separated into the monoatomic layer through washing with a centrifuge using HCl and distilled water.

Accordingly, for example, the alkaline solution may be sodium hydroxide (NaOH), and in the first step, the photocatalyst may be dispersed in the sodium hydroxide through ultrasonic waves, and then the hydrothermal synthesis may be carried out to react the photocatalyst at the temperature of 100°C to 130°C for 15 to 20 hours. In addition, in the first step, after the hydrothermal synthesis, the photocatalyst may be separated into the monoatomic layer through washing with the centrifuge using HCl and distilled water. Alternatively, the alkaline solution may be potassium hydroxide (KOH), and in the first step, the photocatalyst may be dispersed in the potassium hydroxide through ultrasonic waves and then the hydrothermal synthesis may be carried out to react the photocatalyst at the temperature of 100°C to 130°C for 15 to 20 hours. In addition, in the first step, after the hydrothermal synthesis, the photocatalyst may be separated into the monoatomic layer through washing with the centrifuge using HCl and distilled water.

Additionally, the second step may include drying the hydrothermally synthesized photocatalyst.

Additionally, the second step may include calcining the hydrothermally synthesized photocatalyst.

Hereinafter, in order to understand the reforming method according to the present disclosure in more detail, experiments performed in relation to the reforming method according to the present disclosure will be described.

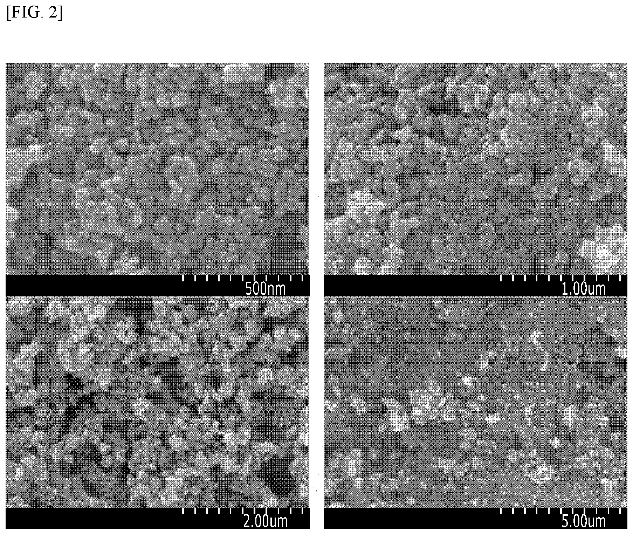

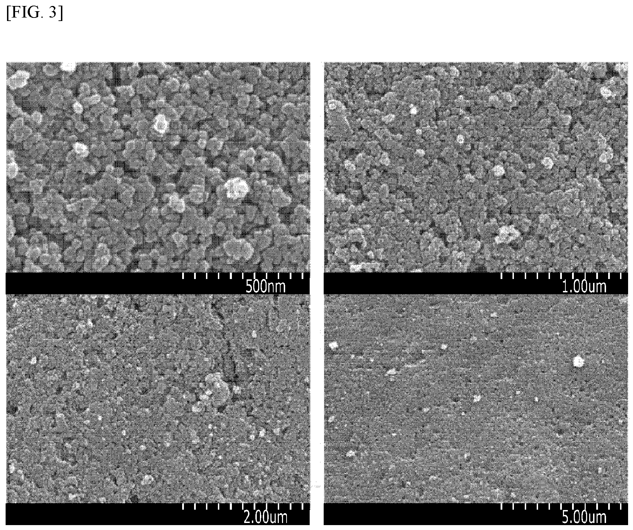

Experiments were performed by the aforementioned reforming method according to the present disclosure, to prepare three types of samples Li-Ti, Na-Ti, and K-Ti, in which a photocatalyst (titanium oxide) was pretreated (reformed) using an alkaline material, and a particle shape of each sample was confirmed through SEM. Observing the particle shapes of the photocatalyst (titanium oxide) before reforming of FIG. 2 and the reformed photocatalyst sample Li-Ti of FIG. 3, it can be seen that the particle composition of the reformed sample is more densely combined than the particle composition of the photocatalyst (titanium oxide) before reforming. In general, in order to increase activity and/or adsorption capacity of a photocatalyst that performs surface reactions, a particle size must be reduced or a dense shape must be formed to increase a specific surface area. In the case of the Li-Ti sample, it can be seen that the density between particles is improved without a significant change in particle size.

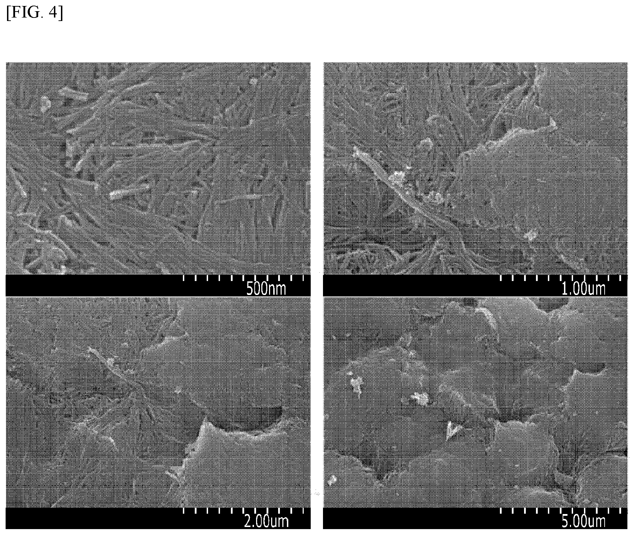

In addition, it can be seen that the particle of the reformed photocatalyst Na-Ti sample of FIG. 4 shows a definite difference from the particle of the photocatalyst (titanium oxide) before reforming. It can be seen that the particle, which was in the form of powder before reforming, changes into a tubular shape and a fibrous tissue is formed.

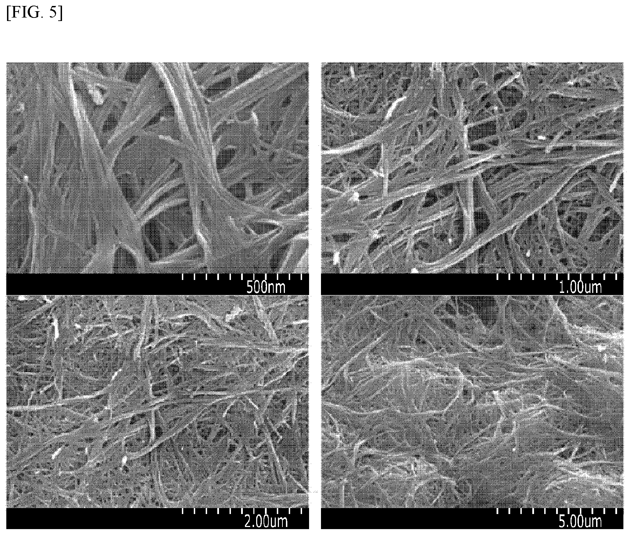

In addition, referring to FIG. 5, it can be confirmed that the reformed photocatalyst K-Ti sample has very smooth tubulous particles and thus exhibits the greatest change in particle shape by the reforming.

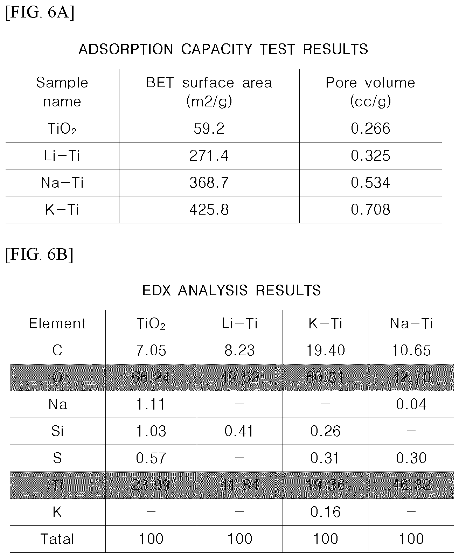

As the purpose of reforming the photocatalyst herein is to improve the activity and adsorption capacity of the photocatalyst, BET analysis was conducted to confirm changes in the photocatalyst before and after reforming. Referring to FIG. 6A, a specific surface area of the photocatalyst (titanium oxide) before reforming was about 59 m²/g. However, it can be seen that after reforming, the specific surface areas of the three types of photocatalyst samples Li-Ti, Na-Ti, and K-Ti all increase. Actually, it can be seen that the specific surface area of the reformed photocatalyst Li-Ti sample increases to about 271 m²/g (4.6 times the specific surface area of the photocatalyst before reforming), the specific surface area of the Na-Ti sample increases to about 369 m²/g (6.2 times the specific surface area of the photocatalyst before reforming), and the specific surface area of the K-Ti sample increases to about 426 m²/g (7.2 times the specific surface area of the photocatalyst before reforming). That is, according to the reforming process of the present disclosure corresponding to a pretreatment step, it can be determined that the reforming process may help improve the activity and/or adsorption capacity of the photocatalyst by virtue of the increase in specific surface area of the photocatalyst even if the photocatalyst does not show a complete nanotubular shape.

In addition, referring to FIG. 6B, according to the results of EXD analysis to determine the elemental composition of the photocatalyst before and after reforming, it can be seen that an amount of titanium oxide contained in the photocatalyst before and after reforming is about 80 to 90%, which means that the content of impurities is not high, but considering that carbon is removed during the calcination process for increasing photocatalytic activity (formation of highly active anatase crystals), the amount of titanium oxide contained in the photocatalyst before reforming is 97%, whereas the amount of titanium oxide contained in each of the three types of photocatalyst samples Li-Ti, Na-Ti, and K-Ti which calcined after reforming is all over 99% (maximum 99.6%), thereby confirming the effect of removing impurities through reforming.

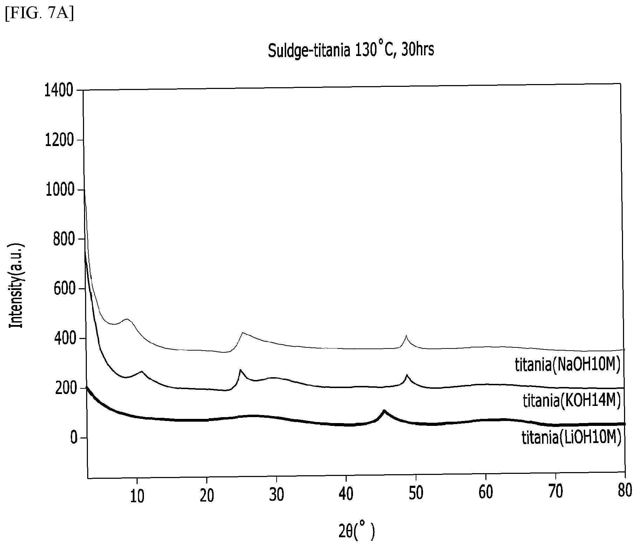

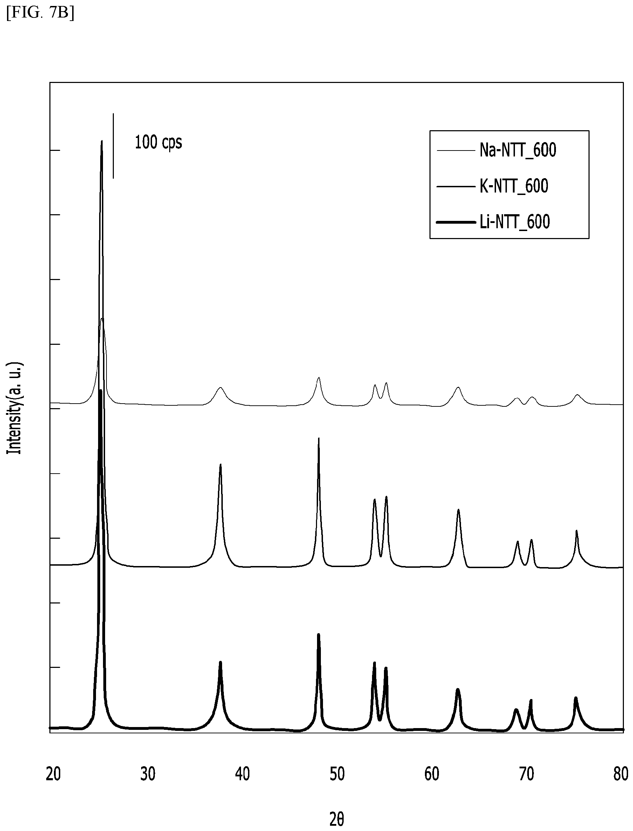

In addition, referring to FIG. 7A, according to the results of analyzing XRD patterns by drying the three types of photocatalyst samples Li-Ti, Na-Ti, and K-Ti reformed using an alkaline material, it can be seen that all the three types of photocatalyst samples are in an amorphous crystal state, and thus a definite anatase peak does not appear. In contrast, referring to FIG. 7B, according to the results of analyzing XRD patterns by calcining the three types of photocatalyst samples at a temperature of 600°C, it can be seen that all the three types of photocatalyst samples have perfect anatase peaks.

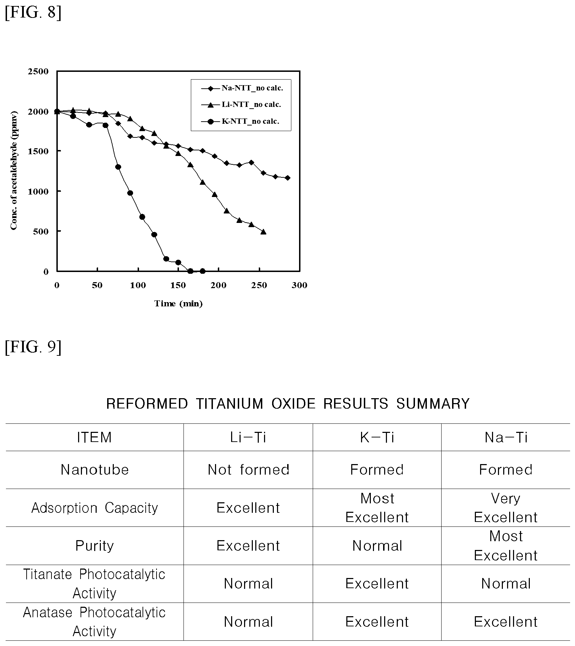

In addition, referring to FIG. 8, when evaluating the organic matter removal effect of the three types of photocatalyst samples, which were reformed by using the alkaline material and then calcined, it can be seen that a significant level of organic matter removal performance is achieved in all the three types of photocatalyst samples, by virtue of the increase in specific surface area, the removal of impurities, and the formation of anatase crystals. In particular, referring to FIG. 9, it can be seen that the photocatalytic activities are greatly improved in K-Ti and Na-Ti as nanotubular anatase crystals are formed through reforming and calcination treatment.

However, the above-described process has advantages of securing a high purity of photocatalyst and increasing the activity and adsorption capacity of the photocatalyst, but has a disadvantage in that manufacturing costs are likely to increase because the treatment process is complicated and many chemicals must be used during the process.

To compensate for this, metatitanic acid (MTA) before crystallization, rather than crystallized photocatalyst (titanium oxide), was reformed by the reforming method according to the present disclosure.

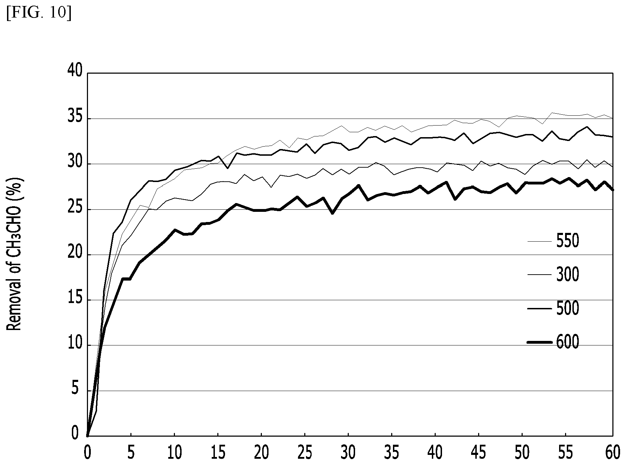

Referring to FIG. 10, it can be seen in view of the organic matter removal effect of three types of samples, which metatitanic acid (MTA) has been reformed with an alkaline material and then calcined at different temperatures, that a sample calcined at 300°C exhibits activity (the organic matter removal effect of about 30%) similar to that of the existing photocatalyst, and a more increased photocatalytic activity is exhibited in a sample calcined at 550°C, but the photocatalytic activity rapidly decreases in the sample calcined at 600°C.

In general, photocatalytic activity is well observed in an anatase shape, anatase crystals can be obtained by calcining the photocatalyst at a temperature of 500 to 600°C, and a temperature at which the anatase shape is transformed into a rutile shape is about 700°C, but it is determined that the change to the rutile shape occurs at a temperature of 600°C when reforming titanic acid (MTA).

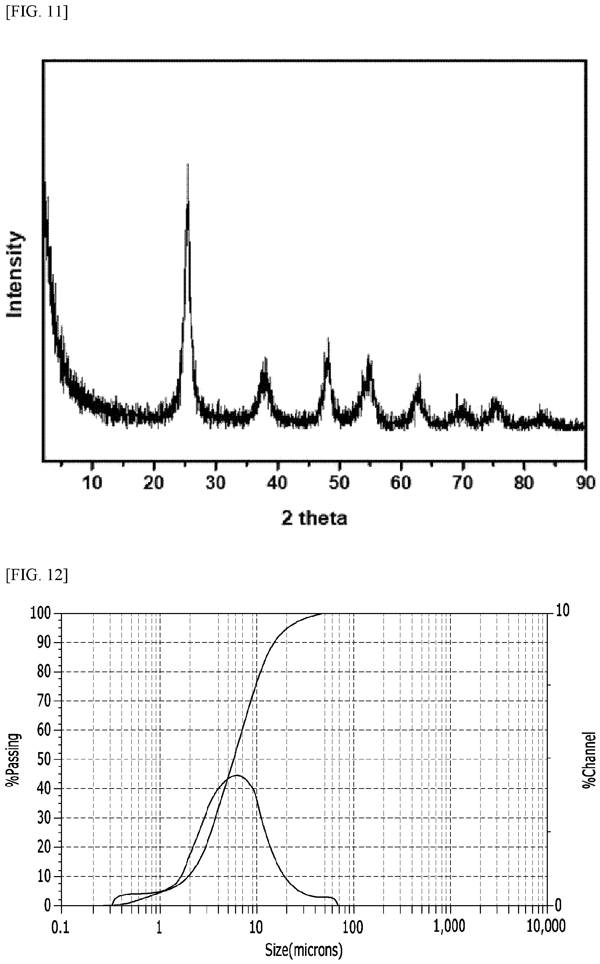

Referring to FIG. 11, it can be seen that an anatase peak appears in the XRD pattern analysis results for the sample (sample calcined at 550°C) confirmed to have the highest photocatalytic activity (organic matter removal effect).

It has been confirmed that the particle size of the photocatalyst that is most easily mixed in the step of producing photocatalyst paste is around 10 µm, and the particle size can be approximated thereto by going through a pulverization process in the photocatalyst preparation step. That is, the reforming method according to the present disclosure may include preparing a photocatalyst before the above-described first step, and the step of preparing the photocatalyst may include pulverizing photocatalyst powder, and the step of pulverizing the photocatalyst powder may be carried out such that an average particle size of the photocatalyst powder is around 10 µm although the particle sizes of the photocatalyst powder have a considerably wide range. Referring to FIG. 12, it can be seen that the photocatalyst powder has been treated to have a particle size distribution of 0.2 to 80 µm and an average particle size of about 8 µm through the step of pulverizing the particles. In addition, in the case of a method for preparing a photocatalyst granular material according to a third embodiment of the present disclosure, which will be described later, a step of preparing a liquid photocatalyst may be performed before a step of coating the granules (granular material) with the liquid photocatalyst. The step of pulverizing the photocatalyst powder may be performed before the step of preparing the liquid photocatalyst.

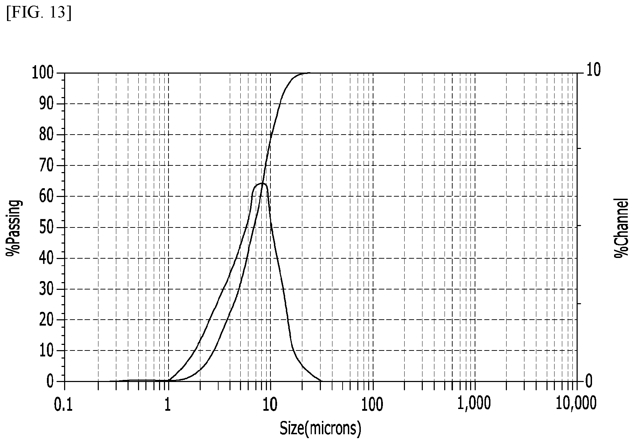

In addition, since photocatalyst particles that are too small or too large may interfere with mixing, the pulverized powder may be classified to more precisely control the distribution of the particles. Referring to FIG. 13, it can be seen that the photocatalyst with a particle size distribution of 0.2 to 80 µm and an average particle size of about 8 µm in FIG. 12 was classified and treated to have a particle size distribution of 1 to 40 µm and an average particle size of about 10 µm.

Summarizing the contents related to the present disclosure based on the experimental results described above, the present disclosure may improve the activity and adsorption capacity through the process of reforming the photocatalyst, namely, the process of increasing the specific surface area of the photocatalyst (titanium oxide) particles and removing impurities. For this purpose, in the reforming method of the present disclosure, the photocatalyst (titanium oxide) powder may be hydrothermally synthesized in a high-temperature alkaline solution to be separated into a monoatomic layer and then dried, thereby forming a highly pure tubular crystal from which impurities have been removed. Actually, according to the aforementioned experimental results, it was confirmed that the three types of samples Li-Ti, Na-Ti, and K-Ti were prepared by treating the photocatalyst with the high-temperature alkaline solution, and in this case, the Li-Ti sample has improved inter-particle density (see FIG. 3), and the NaTi sample, and K-Ti sample had the particles changed into the tubular shape and could form the fibrous tissue (see FIGS. 4 and 5).

In addition, according to the aforementioned experimental results, it was confirmed through BET analysis that the specific surface area increased for all the three types of photocatalysts treated (reformed) with the high-temperature alkaline solution more than that of the photocatalyst before reforming (increased by 4.6 times for the Li-Ti sample, 6.2 times for the Na-Ti sample, and 7.2 times for the K-Ti sample), it was confirmed through EXD analysis that the content rate of titanium oxide increased for all the three types of photocatalysts reformed with the alkaline solution, and it was confirmed through XRD pattern analysis that the anatase crystals were formed in the photocatalyst calcined at 600°C. Also, it was confirmed through the organic matter removal experiment that the reformed and calcined photocatalyst exhibited superior removal performance by virtue of the improved activity and adsorption capacity.

In addition, it was confirmed that the results of pretreatment (reforming) of the photocatalyst using the alkaline material were good, but the treatment process was complicated and the manufacturing cost might increase. To compensate for this, it was confirmed that metatitanic acid (MTA) before crystallization, other than crystallized photocatalyst (titanium oxide), could be reformed by the reforming method according to the present disclosure.

The metatitanic acid (MTA) may be reformed through the photocatalytic reforming method described above, namely, through the method of dispersing the photocatalyst in the alkaline solution such as sodium hydroxide (NaOH) or potassium hydroxide (KOH) through ultrasonic waves, followed by the hydrothermal synthesis at a temperature of 100°C to 130°C for 15 to 20 hours, to separate the photocatalyst into the monoatomic layer. In addition, the metatitanic acid (MTA) may calcine at a temperature of 300°C to 550°C (preferably 550°C) to maximize activity and/or adsorption capacity.

In addition, as described above, the reforming method according to the present disclosure may include preparing the photocatalyst before the hydrothermal synthesis step, and the step of preparing the photocatalyst may include preparing the photocatalyst powder. In the step of preparing the photocatalyst powder, the photocatalyst powder may be prepared using an acid-fast intermediate. The effects of the step of preparing the photocatalyst powder in the reforming method according to the present disclosure will be described later. The step of preparing the photocatalyst powder may be performed before the step of pulverizing the photocatalyst powder described above.

Additionally, the present disclosure provides a photocatalyst according to one embodiment of the present disclosure. The photocatalyst according to one embodiment of the present disclosure includes a plurality of photocatalyst crystals reformed by a photocatalytic reforming method according to one embodiment of the present disclosure.

Additionally, the present disclosure provides a photocatalyst granular material (or granule) according to a first embodiment of the present disclosure. The photocatalyst granular material according to the first embodiment of the present disclosure includes a photocatalyst (reformed photocatalyst) containing a plurality of photocatalyst crystals reformed by a photocatalytic reforming method.

Additionally, the photocatalyst including a plurality of reformed photocatalyst crystals includes a plurality of photocatalyst crystals reformed by the photocatalytic reforming method according to one embodiment of the present disclosure described above.

Additionally, the present disclosure provides a method for preparing a photocatalyst granular material according to one embodiment of the present disclosure. Hereinafter, a method for preparing a photocatalyst granular material according to a first embodiment of the present disclosure (hereinafter, referred to as 'a first photocatalyst granular material preparation method of the present disclosure') will be described. However, the first photocatalyst granular material preparation method of the present disclosure may use a photocatalyst reformed by the photocatalytic reforming method according to one embodiment of the present disclosure described above, and may include the photocatalytic reforming method according to one embodiment of the present disclosure described above. Therefore, redundant descriptions of the photocatalytic reforming method according to one embodiment of the present disclosure and the photocatalyst granular material according to the first embodiment of the present disclosure described above will be briefly given or omitted.

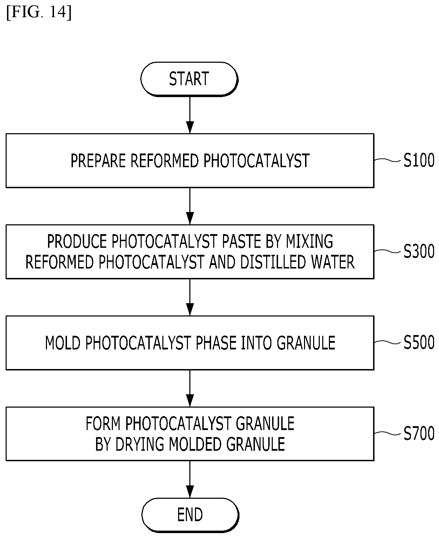

Referring to FIG. 14, the first preparation method of the present disclosure includes preparing a photocatalyst reformed by the photocatalytic reforming method according to one embodiment of the present disclosure described above (S100). The reformed photocatalyst may include tubular crystals. Additionally, the tubular crystals may be formed by performing hydrothermal synthesis between photocatalyst powder and an alkaline solution, separating the monoatomic layer, and then rolling the monoatomic layer.

Additionally, referring to FIG. 14, the first preparation method includes producing a photocatalyst paste by mixing the reformed photocatalyst and distilled water (S300). For example, the reformed photocatalyst and the distilled water may be mixed at a composition ratio of 75 : 25. Additionally, in step S300, the paste may be produced while adding the distilled water to the reformed photocatalyst.

Additionally, referring to FIG. 14, the first preparation method includes forming (molding) the photocatalyst paste into a granular shape (S500). In step S500, the photocatalyst paste may be formed in a granular shape by putting it into a pill maker. In step S500, the granule may be molded (formed) by setting its size in consideration of the fact that the molded granule undergoes drying shrinkage during step 5700 (drying step) to be explained later (for example, it may shrink by about 10% of a volume). In other words, in step S500, considering that the size of the granule is reduced in step 5700, the granule may be formed to be greater than a finally prepared photocatalyst granular material (photocatalyst granule after drying). Accordingly, the mold of the pill maker may be manufactured by taking into account that the size of the granular material is reduced by about 10% after drying.

For reference, in the present disclosure, the granule (granular material) may encompass various shapes such as sphere (ball shape), square, triangle, pellet, hexahedron, and the like. Accordingly, the photocatalyst granular material may be formed in various shapes such as sphere, square, triangle, pellet, hexahedron, and the like.

Additionally, the molding conditions of the pill maker may be set so that the photocatalyst paste may be released from the mold and discharged in the granular shape.

Additionally, the molded granule may contain moisture.

Additionally, referring to FIG. 14, the first preparation method includes drying the molded granule to form a photocatalyst granular material (granule) (S700). In step S700, the moisture-containing bead (ring)-shaped granule formed in step S500 may be dried to be completely made as a photocatalyst granule. At this time, the photocatalyst granule completely made through step S700 must have a certain hardness in order to be used for various purposes to remove harmful substances (fine dust precursors, volatile organic compounds) and harmful microorganisms (viruses or bacteria), etc., and powder should not be separated from the surface of the granule. Step S700 may be performed to prepare such photocatalyst granules.

Additionally, in step 5700, the granules may be dried using a drying furnace. A non-continuous type drying furnace (batch type furnace) or a continuous type drying furnace (continuous type furnace) may be used as the drying furnace. Non-continuous type drying refers to a method in which products are loaded and operated in cycle units, namely, the products remain inside the drying furnace until heat treatment is completed, whereas continuous type drying refers to a method in which heat treatment conditions of products, such as temperature profile, oxygen concentration, etc. are set and the products are directly moved and thermally treated according to the set conditions.

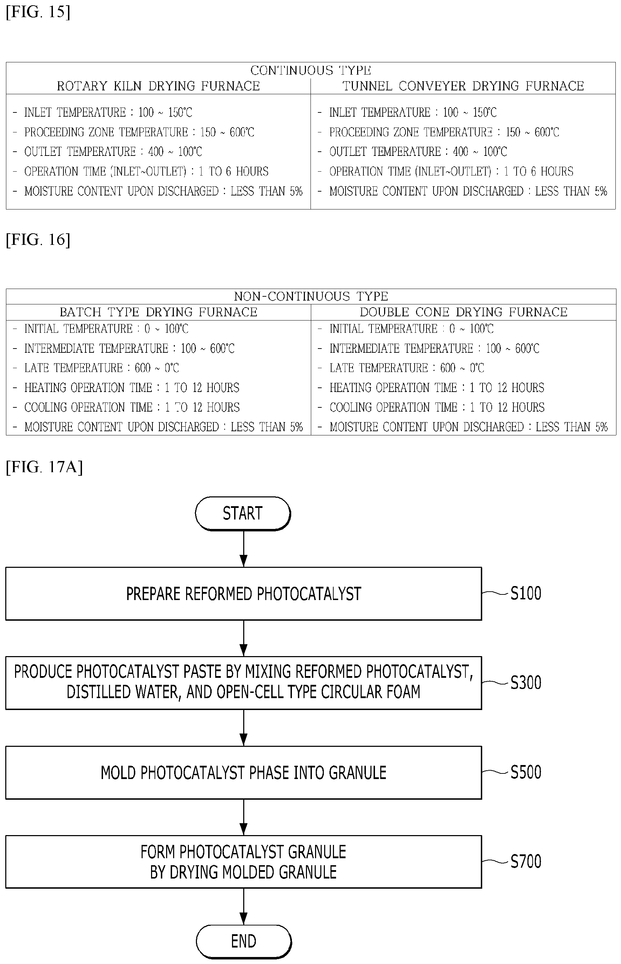

Heating conditions (performance conditions of step S700) according to the drying furnace type in the case of the continuous type drying furnace are shown in FIG. 15, and heating conditions according to the drying furnace type in the case of the non-continuous type drying furnace are shown in FIG. 16, respectively.

In addition, the present disclosure provides a photocatalyst granular material according to a second embodiment of the present disclosure (hereinafter, referred to as 'a second photocatalyst granular material of the present disclosure'). The second photocatalyst granular material of the present disclosure may be prepared by the photocatalyst granular material preparation method according to the first embodiment of the present disclosure described above. Therefore, redundant descriptions of the photocatalytic reforming method according to one embodiment of the present disclosure, the photocatalyst granular material according to the first embodiment of the present disclosure, and the photocatalyst granular material preparation method according to the first embodiment of the present disclosure will be briefly given or omitted.

In the second photocatalyst granular material of the present disclosure, the photocatalyst particle forms a tubular crystal.

The tubular crystal may be produced by separating the monoatomic layer through the hydrothermal synthesis of the photocatalyst and the alkaline solution.

Hereinafter, a method for preparing a photocatalyst granular material according to a second embodiment of the present disclosure (hereinafter, referred to as 'a second preparation method of the present disclosure') will be described. However, in a description related to the second preparation method of the present disclosure, redundant descriptions of the photocatalytic reforming method according to one embodiment of the present disclosure, the photocatalyst granular material preparation method according to the first embodiment of the present disclosure, or the photocatalyst granular materials according to the first and second embodiments of the present disclosure will be briefly given or omitted.

The second preparation method of the present disclosure may be applied to molding of photocatalyst granules to ensure porosity. If the photocatalyst granules are porous, decomposition/oxidation and adsorption capacity for gaseous harmful substances may be strengthened.

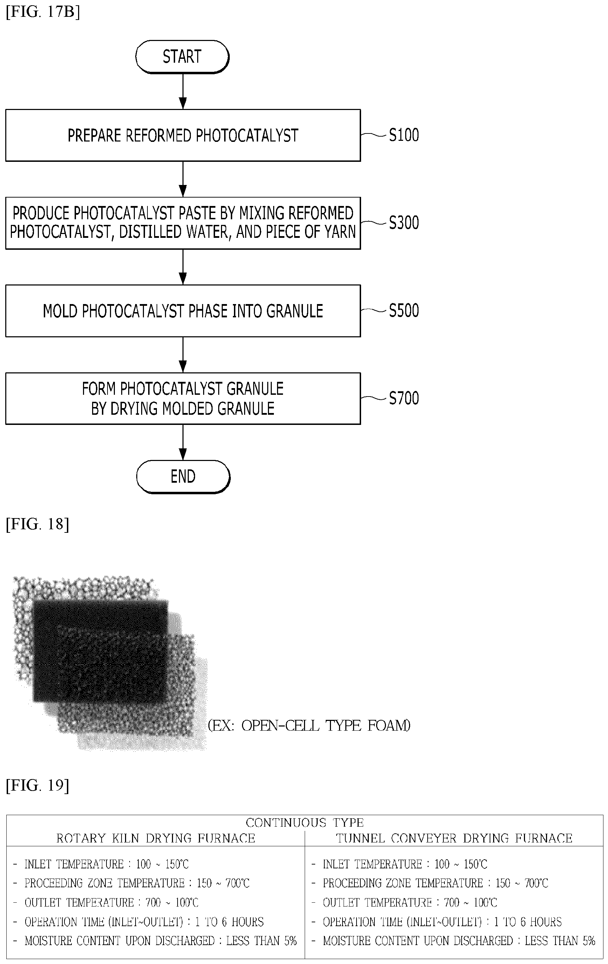

Referring to FIGS. 17A and 17B, the second preparation method of the present disclosure includes preparing a photocatalyst reformed by the photocatalytic reforming method according to one embodiment of the present disclosure described above (S100). The reformed photocatalyst may include tubular crystals. For example, in step S 100, the photocatalyst powder and the alkaline solution may be hydrothermally synthesized to separate the monoatomic layer, thereby pretreating the crystal of the photocatalyst powder to have a tubular shape. For example, in step S 100, the photocatalyst powder may be separated into the monoatomic layer by undergoing hydrothermal synthesis in the alkaline solution at a high temperature of at least 100°C for a long time, and then the photocatalyst crystal may be transformed into a tubular shape. Here, the alkaline solution may be sodium hydroxide (NaOH).

More specifically, in step S 100, a photocatalyst with an anatase phase may be dispersed in sodium hydroxide through ultrasonic waves, followed by hydrothermal synthesis to react the photocatalyst at a temperature of 100°C to 130°C for 15 to 20 hours, and then separated into a monoatomic layer through washing with a centrifuge using HCl and distilled water. The separated monoatomic layer may be rolled and transformed into a tubular shape. Additionally, in step S 100, drying or calcination may be performed so that the monoatomic layer is transformed into the tubular shape. Since this has been described in detail in the photocatalytic reforming method described above, a detailed description thereof will be omitted.

Additionally, referring to FIGS. 17A and 17B, the second preparation method of the present disclosure may include producing a photocatalyst paste by mixing the reformed photocatalyst and distilled water. In the step of producing the photocatalyst paste, the photocatalyst paste may be produced by additionally mixing an open-cell type circular foam made of a material with a lower melting point than that of the reformed photocatalyst or a piece of yarn (yarn piece) made of a material with a lower melting point than that of the reformed photocatalyst. That is, the second preparation method of the present disclosure includes mixing the reformed photocatalyst powder and distilled water, and producing a photocatalyst paste by additionally mixing an open-cell type circular foam made of a material with a lower melting point than that of the reformed photocatalyst or a piece of yarn made of a material with a lower melting point than that of the reformed photocatalyst (S300). For example, referring to FIG. 17A, in step S300, the paste may be produced while adding the circular foam and distilled water to the reformed photocatalyst powder. Alternatively, referring to FIG. 17B, in step S300, the paste may be produced while adding the piece of yarn and distilled water to the reformed photocatalyst powder. Accordingly, in step 5700, which will be described later, the circular foam or piece of yarn may be at least partially melted so as to form pores, and the photocatalyst granule may have porosity.

The circular foam may be made of a material containing at least one of polyethylene and polyurethane. However, the material forming the circular foam is not limited to this, and a material with a lower melting point than that of the photocatalyst may form the circular foam. Additionally, the piece of yarn may be made of a material containing at least one of polyethylene and polyurethane. However, the material forming the piece of yarn is not limited to this, and a material with a lower melting point compared to the photocatalyst may form the piece of yarn.

For reference, FIG. 18 shows an example of an open-cell type foam.

Additionally, referring to FIGS. 17A and 17B, the second preparation method of the present disclosure includes forming (molding) the photocatalyst paste into the granular shape (S500). In step S500, the photocatalyst paste may be molded into a granular shape by putting it into a pill maker. In step S500, the granule may be molded by setting its size in consideration of the fact that the molded granule undergoes drying shrinkage during step 5700 (drying step) to be explained later (for example, it may shrink by about 10% of a volume). In other words, in step S500, considering that the size of the granule is reduced in step 5700, the granule may be molded to be greater than a finally prepared photocatalyst granular material (photocatalyst granule after drying). Accordingly, the mold of the pill maker may be manufactured by taking into account that the size of the granular material is reduced by about 10% after drying.

Additionally, the molding conditions of the pill maker may be set so that the photocatalyst paste may be released from the mold and discharged in the granular shape.

Additionally, the molded granule may contain moisture.

Additionally, referring to FIGS. 17A and 17B, the second preparation method of the present disclosure includes drying the molded granule to form a photocatalyst granule (S700).

In step S700, the moisture-containing bead (ring)-shaped granule formed in step S300 may be dried to be completely made as a photocatalyst granule. At this time, the photocatalyst granule completely made through step S700 must have a certain hardness in order to be used for various purposes to remove harmful substances (fine dust precursors, volatile organic compounds) and harmful microorganisms (viruses or bacteria), etc., and powder should not be separated from the surface of the granule. Step S700 may be performed to prepare such photocatalyst granules.

Additionally, in step 5700, the molded granule may be dried at a temperature higher than the melting point of the circular foam or yarn piece so that at least a portion of the circular foam or yarn piece is melted. In other words, step S700 may be performed so that at least a portion of the circular foam is melted when the photocatalyst paste contains the circular foam. Additionally, step S700 may be performed so that at least a portion of the piece of yarn is melted when the photocatalyst paste contains the piece of yarn. Accordingly, the circular foam or piece of yarn may be melted so as to form pores, and the photocatalyst granule may have porosity.

Additionally, in step 5700, the granule may be dried using a drying furnace. A non-continuous type drying furnace (batch type furnace) or a continuous type drying furnace (continuous type furnace) may be used as the drying furnace. Non-continuous type drying refers to a method in which products are loaded and operated in cycle units, namely, the products remain inside the drying furnace until heat treatment is completed, whereas continuous type drying refers to a method in which heat treatment conditions of products, such as temperature profile, oxygen concentration, etc. are set and the products are directly moved and thermally treated according to the set conditions.

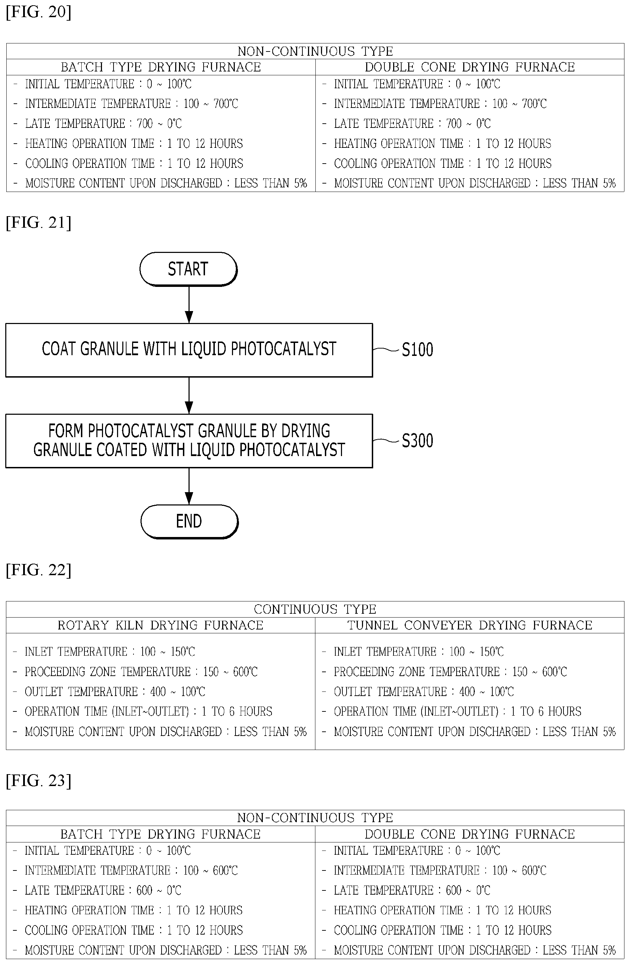

Heating conditions (performance conditions of step S700) according to the drying furnace type in the case of the continuous type drying furnace are shown in FIG. 19, and heating conditions according to the drying furnace type in the case of the non-continuous type drying furnace are shown in FIG. 20, respectively.

Additionally, the present disclosure provides a photocatalyst granular material according to a third embodiment of the present disclosure. The photocatalyst granular material according to the third embodiment of the present disclosure is prepared by the photocatalyst granular material preparation method according to the second embodiment of the present disclosure. The photocatalyst granular material according to the third embodiment of the present disclosure includes tubular crystals formed by the reformed photocatalyst powder and pores formed between at least some of the crystals. The pores are formed by drying the granule prepared by molding the photocatalyst paste, which has been prepared by adding, to the reformed photocatalyst powder and the distilled water, the open-cell type circular foam made of a material with a lower melting point than that of the photocatalyst powder (reformed photocatalyst powder) or the piece of yarn made of a material with a lower melting point than that of the photocatalyst (reformed photocatalyst powder), at a temperature higher than the melting point of the circular foam or piece of yarn such that the circular foam or piece of yarn is at least partially melted.

In addition, the tubular crystal may be produced by separating the monoatomic layer through the hydrothermal synthesis of the photocatalyst and the alkaline solution.

Hereinafter, a method for preparing a photocatalyst granular material according to a third embodiment of the present disclosure (hereinafter, referred to as 'a third preparation method of the present disclosure') will be described. However, in a description related to the third preparation method of the present disclosure, redundant descriptions of the photocatalyst granular material preparation methods according to the first and second embodiments of the present disclosure or the photocatalyst granular materials according to the first to third embodiments of the present disclosure will be briefly given or omitted.

Referring to FIG. 21, the third preparation method of the present disclosure includes coating a granule with a liquid photocatalyst at room temperature (S100). The granule is a material that does not contain a photocatalyst. For example, the granule may be made of a material containing at least one of ceramic, metal, glass, etc.

In step S 100, the granule is immersed in the liquid photocatalyst or the granule is sprayed with the liquid photocatalyst.

When the granule is coated with the liquid photocatalyst using the immersion method in step S 100, the granule made of a material such as ceramic, metal, and glass to be coated may be immersed into a container containing the liquid photocatalyst at room temperature, so that the liquid photocatalyst may be evenly distributed on the granule.

In addition, when the granule is coated with the liquid photocatalyst using the spraying method in step S 100, the liquid photocatalyst may be applied by being sprayed on a material such as ceramic, metal, or glass to be coated at room temperature.

Additionally, referring to FIG. 21, the third preparation method of the present disclosure includes forming a photocatalyst granule by drying the granule coated with the liquid photocatalyst (S300). The photocatalyst granule completely formed through step S300 must have a certain hardness in order to be used for various purposes to remove harmful substances (fine dust precursors, volatile organic compounds) and harmful microorganisms (viruses or bacteria), etc., and the photocatalyst should not be separated from the surface of the granule. Step S300 may be performed to prepare such photocatalyst granules. The liquid photocatalyst includes a photocatalyst reformed by the reforming method of the present disclosure described above.

Additionally, in step S300, the granule may be dried using a drying furnace. A non-continuous type drying furnace (batch type furnace) or a continuous type drying furnace (continuous type furnace) may be used as the drying furnace. Non-continuous type drying refers to a method in which products are loaded and operated in cycle units, namely, the products remain inside the drying furnace until heat treatment is completed, whereas continuous type drying refers to a method in which heat treatment conditions of products, such as temperature profile, oxygen concentration, etc. are set and the products are directly moved and thermally treated according to the set conditions.

Heating conditions (performance conditions of step S300) according to the drying furnace type in the case of the continuous type drying furnace are shown in FIG. 22, and heating conditions according to the drying furnace type in the case of the non-continuous type drying furnace are shown in FIG. 23, respectively.

Additionally, the present disclosure provides a photocatalyst granular material according to a fourth embodiment of the present disclosure. The photocatalyst granular material according to the fourth embodiment of the present disclosure is prepared by the photocatalyst granular material preparation method according to the third embodiment of the present disclosure.

This fourth photocatalyst granular material of the present disclosure includes a granule. The granule may be made of a material containing at least one of ceramic, metal, glass, etc.

Additionally, the fourth photocatalyst granular material of the present disclosure includes a photocatalyst coating layer formed on a surface of the granule and made of a photocatalyst. The photocatalyst coating layer is formed by immersing the granule in a liquid photocatalyst or spraying the liquid photocatalyst on the granule.

The photocatalyst and photocatalyst granular material prepared by the photocatalyst granular material preparation methods according to the above-described first to fourth embodiments may have virus or bacteria removal performance.

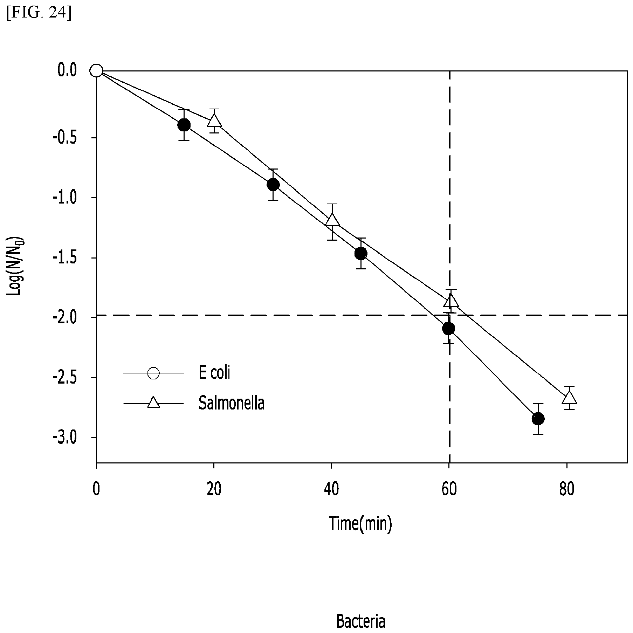

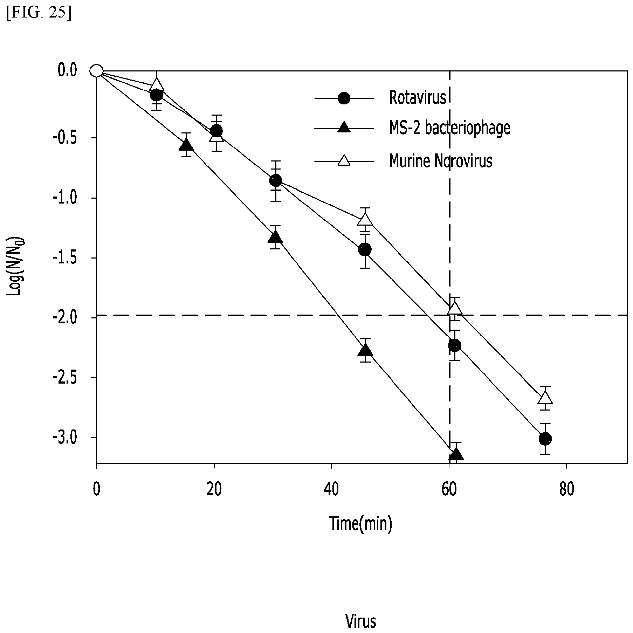

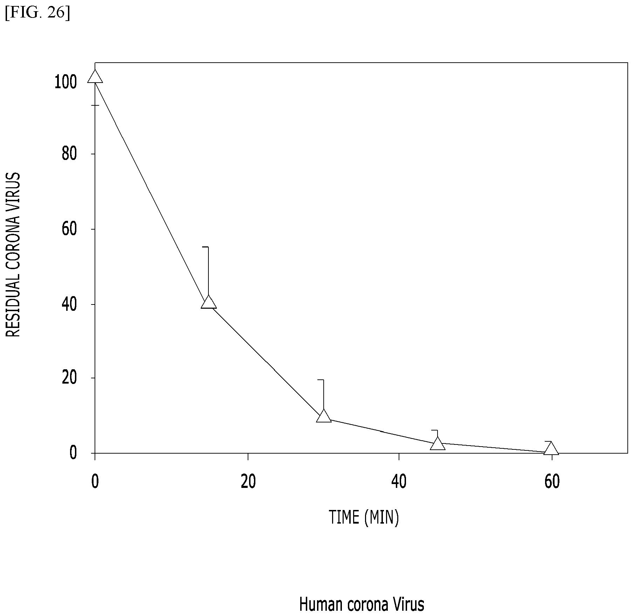

Referring to FIG. 24, the photocatalyst before reforming can remove 99% of bacteria such as *E. coli*, *Salmonella*, etc. in 60 minutes. In addition, referring to FIGS. 25 and 26, it can be confirmed that the photocatalyst before reforming has removal performance of 99% for various viruses, such as Noro Virus, Rota Virus, Bacteriophage MS2, Human Coronavirus (HcoV), etc. in 60 minutes. In other words, the photocatalyst can remove bacteria and viruses regardless of their types, and especially, can continuously remove microorganisms to be removed as time passes.

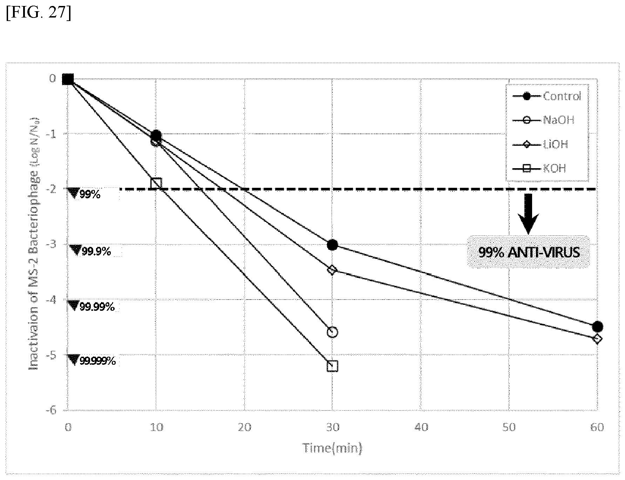

In addition, as the photocatalyst constituting the granular material according to the present disclosure, the virus removal performance of the three types of photocatalyst samples Li-Ti, Na-Ti, and K-Ti, which have been reformed using the alkaline material according to the above-described reforming method of the present disclosure, can be greatly improved compared to the photocatalyst before reforming (control). In fact, referring to FIG. 27, it can be confirmed that the photocatalyst before reforming exhibits virus removal performance at the level of 99.9% in 30 minutes. In contrast, it can be confirmed that the three types of photocatalyst samples Li-Ti, Na-Ti, and K-Ti exhibit high removal performance of up to 99.9994% or more in 30 minutes (99.96% for Li-Ti, 99.997% for Na-Ti, and 99.9994% for K-Ti).

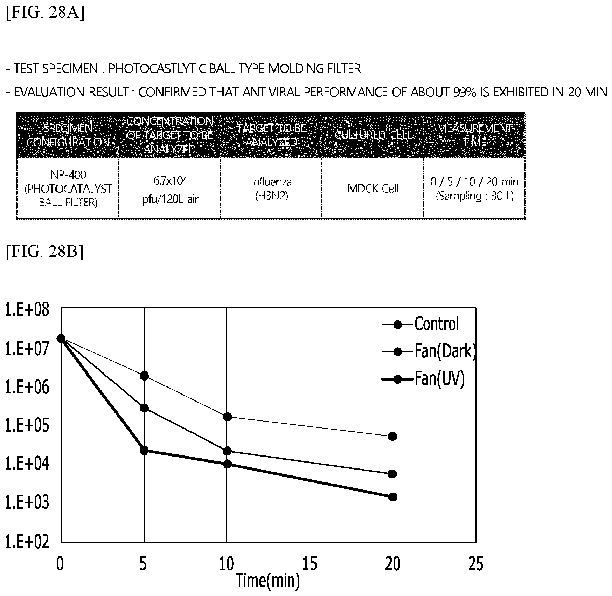

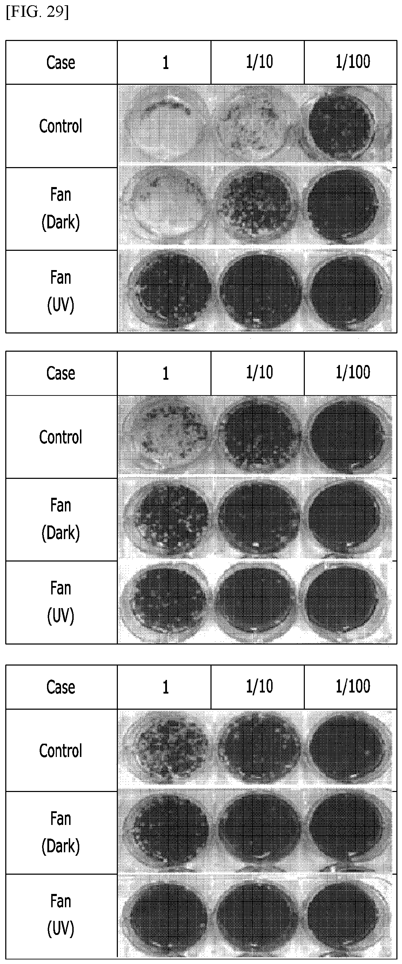

In addition, referring to FIGS. 28A, 28B, and 29, in the case of the photocatalyst molded granule (more precisely, a non-filtering type air purification filter with casing plural photocatalyst granules), it can be confirmed that about 99% of influenza viruses are removed in 20 minutes.

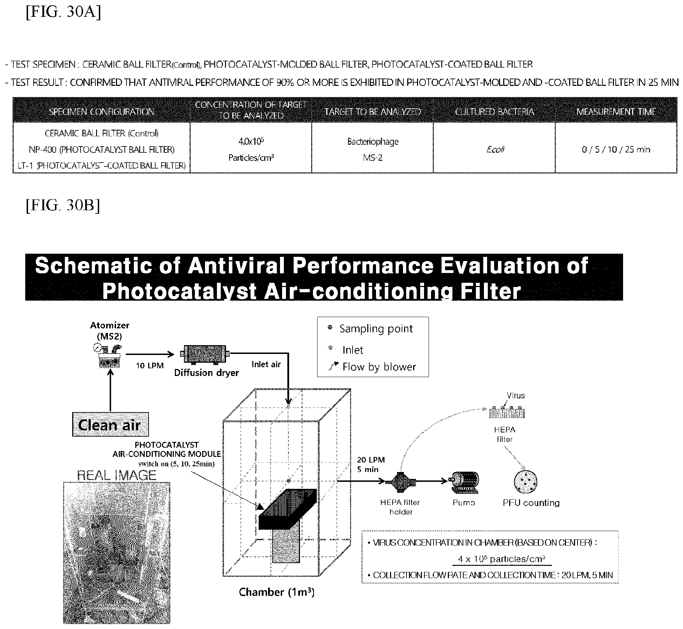

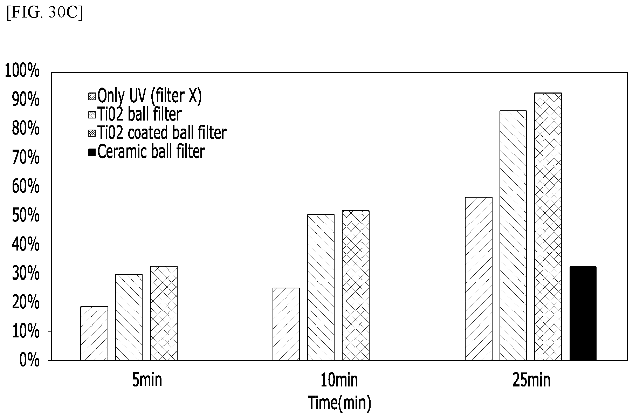

In addition, the virus removal performance of the photocatalyst molded granule and the photocatalyst coated granule may exhibit significant antiviral performance compared to filters (ceramic filters) without photocatalysts applied. In fact, referring to FIGS. 30A to 30C, it can be seen that the virus removal performance of the ceramic granule is minimal, but the photocatalyst granule exhibits excellent virus removal performance of about 90% in 25 minutes.

Additionally, in the present disclosure, the photocatalyst or photocatalyst powder may contain titanium oxide. When the photocatalytic reforming method according to one embodiment of the present disclosure as described above is applied to titanium oxide, various impurities contained in titanium oxide crystals may be removed and simultaneously the tubular crystals may be formed, thereby increasing the adsorption capacity of the powder itself, which may result in a synergistic effect in photocatalyst efficiency.

According to the photocatalytic reforming method according to one embodiment of the present disclosure, titanium oxide powder may be reacted in a high-temperature alkaline solution for a long time, and accordingly, the titanium oxide powder may be separated into a monoatomic layer by the alkaline solution. Also, the separated monoatomic layer may be rolled into a tubular shape. In the process of dissolution by the alkaline solution, impurities contained in the titanium oxide may be removed, and only pure titanium oxide may be recovered. In addition, in the step of pretreating the photocatalyst powder (reforming process), general titanium oxide may be added and mixed and stirred with the alkaline solution, to perform reaction therebetween. At this time, the hydrothermal synthesis may be carried out while maintaining a temperature of a reactor at 130°C.

Also, for reference, in the present disclosure, as described above, the reforming method may include the step of preparing the photocatalyst powder.

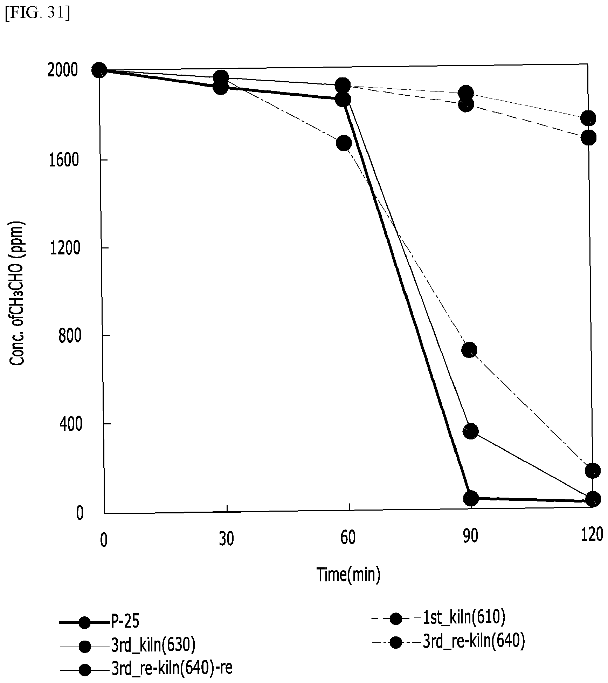

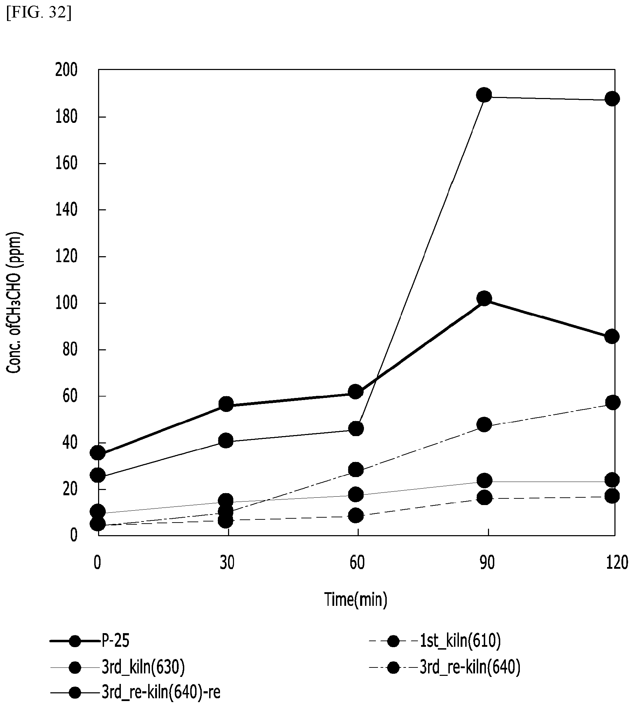

In the step of preparing the photocatalyst powder, the photocatalyst powder may be prepared using an acid-fast intermediate. Referring to FIGS. 31 and 32, the prepared photocatalyst powder may exhibit photocatalytic activity at a calcination temperature of 600°C or higher, but the photocatalytic activity may significantly decrease after 700°C. In addition, the activity of a photocatalyst sample retreated at a calcination temperature of 640°C may be excellent.

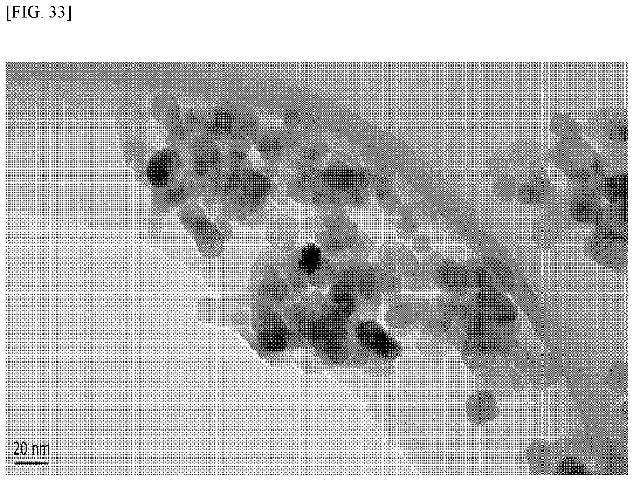

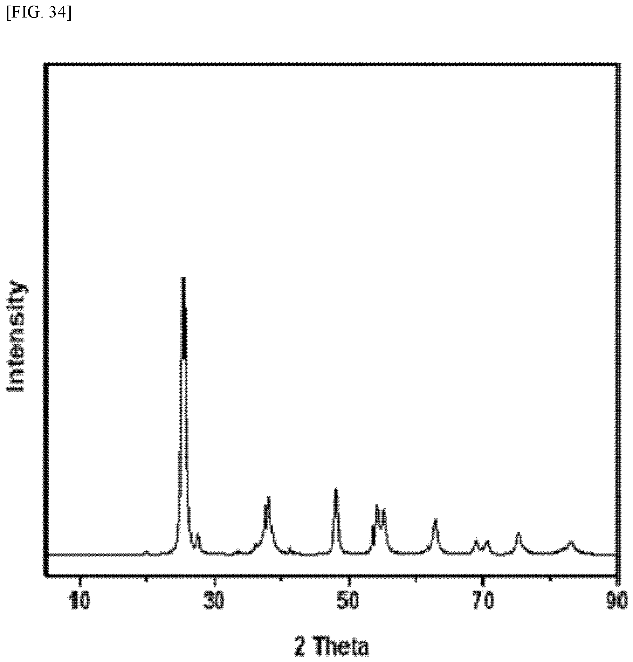

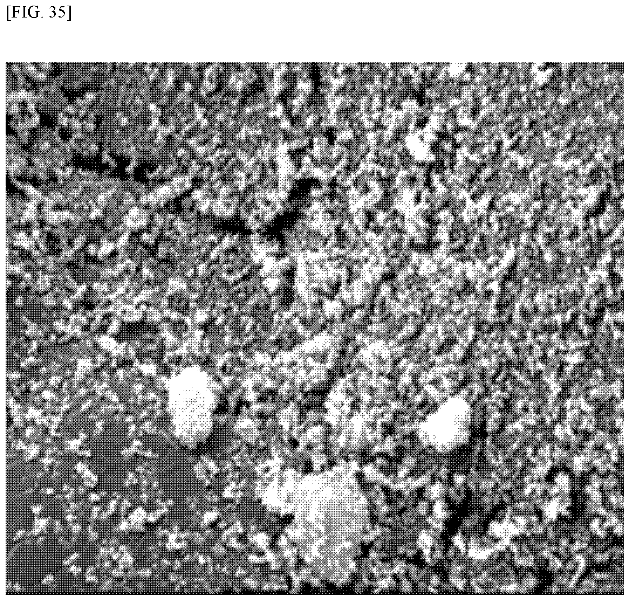

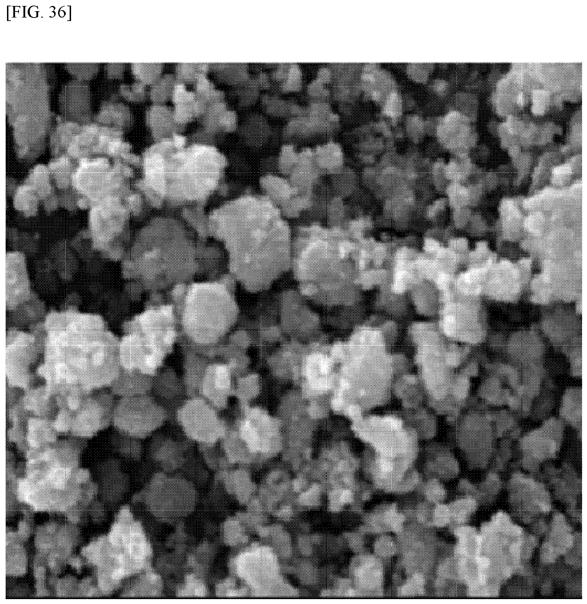

For reference, FIG. 33 shows a TEM measurement photo of the photocatalyst powder prepared by the step of preparing the photocatalyst powder of the present disclosure, FIG. 34 shows XRD measurement results of the photocatalyst powder prepared by the step of preparing the photocatalyst powder of the present disclosure, FIG. 35 shows an SEM/EDX (X1,300) photo of the photocatalyst powder prepared by the step of preparing the photocatalyst powder of the present disclosure, and FIG. 36 shows an SEM/EDX (X10,000) photo of the photocatalyst powder prepared by the step of preparing the photocatalyst powder of the present disclosure.

The foregoing description of the present disclosure is for illustrative purposes only, and it will be understood by those of ordinary skill in the art that the present disclosure can be easily modified into other specific forms without changing technical idea or essential features of the present disclosure. Therefore, the embodiments described above should be understood in all aspects as illustrative and not limited. For example, each component described as a singular form may be implemented in a distributed manner, and similarly, components described as distributed may also be implemented in a combined form.

The scope of the present disclosure is determined by the claims to be described later rather than the detailed description above. All changes or modifications derived from the meaning and scope of the claims and their equivalent concepts should be construed as being included in the scope of the present disclosure.

## Claims

1. A photocatalytic reforming method comprising:
performing hydrothermal synthesis to react a photocatalyst with an alkaline solution at a temperature of 100°C or higher, so that a monoatomic layer of the photocatalyst is separated; and
treating the hydrothermally synthesized photocatalyst so that a crystal of the photocatalyst has a tubular shape.

2. The photocatalytic reforming method of claim 1, wherein the performing the hydrothermal synthesis is to react the photocatalyst at a temperature of 100°C to 130°C by mixing the photocatalyst with the alkaline solution.

3. The photocatalytic reforming method of claim 1, wherein the alkaline solution contains at least one of Li, Na, and K.

4. The photocatalytic reforming method of claim 1, wherein the photocatalyst has an anatase phase.

5. The photocatalytic reforming method of claim 1, wherein the treating includes drying the hydrothermally synthesized photocatalyst.

6. The photocatalytic reforming method of claim 1, wherein the treating includes calcining the hydrothermally synthesized photocatalyst.

7. The photocatalytic reforming method of claim 1, wherein the alkaline solution contains at least one of Na and K, and the photocatalyst has an anatase phase.

8. The photocatalytic reforming method of claim 7, wherein the alkaline solution contains at least one of NaOH and KOH, and the performing the hydrothermal synthesis is to perform the hydrothermal synthesis by dispersing the photocatalyst with the anatase phase in the alkaline solution through energy equivalent to ultrasonic waves, and then reacting the photocatalyst with the alkaline solution at a temperature of 100°C to 130°C for 15 to 20 hours.

9. The photocatalytic reforming method of claim 1, wherein the photocatalyst is titanium oxide or metatitanic acid.

10. A photocatalyst granular material comprising a photocatalyst having a plurality of photocatalyst crystals reformed by the photocatalytic reforming method according to claim 1.

11. A photocatalyst granular material preparation method comprising:
preparing a photocatalyst reformed by the photocatalytic reforming method according to claim 1;
producing a photocatalyst paste by mixing the reformed photocatalyst with distilled water;
molding the photocatalyst paste into a granule; and
drying the molded granule to form a photocatalyst granule.

12. The photocatalyst granular material preparation method of claim 11, wherein the producing the photocatalyst paste is to further mix an open-cell type circular foam made of a material with a lower melting point than that of the reformed photocatalyst or a piece of yarn made of a material with a lower melting point than that of the photocatalyst.

13. The photocatalyst granular material preparation method of claim 12, wherein the drying the molded granule is to dry the molded granule at a temperature higher than the melting point of the circular foam or the piece of yarn, such that at least a portion of the circular foam or piece of yarn is melted.

14. The photocatalyst granular material preparation method of claim 12, wherein the circular foam or the piece of yarn is made of a material containing at least one of polyethylene and polyurethane.

15. A photocatalyst granular material preparation method comprising:
coating a granule with a liquid photocatalyst; and
drying the granule coated with the liquid photocatalyst to form a photocatalyst granule,
wherein the coating of the granule with the liquid photocatalyst is to immerse the granule in the liquid photocatalyst or spray the liquid photocatalyst on the granule, the liquid photocatalyst includes a photocatalyst reformed by the photocatalytic reforming method according to claim 1, and the granule is a material that does not contain the photocatalyst.
